# EUROPÄISCHE PATENTANMELDUNG

(11) **EP 2 764 920 A2**
(43) Veröffentlichungstag der Anmeldung: **13.08.2014**
(21) Anmeldenummer: 14153370.3
(22) Anmeldetag: 31.01.2014
(51) Int. Cl.: B01L 7/00

(54) **Kühlbox mit einem mit röhrchenförmigen Gefäßen bestückten Rack zum automatischen Befüllen mit einem Pipettierautomaten**

(30) Priorität: 06.02.2013 DE 102013101176
(71) Anmelder: CyBio AG, 07745 Jena (DE)
(72) Erfinder: Moore, Thomas, 07751 Jena (DE); Kiehntopf, Dr. Dr. Michael, 07743 Jena (DE)
(74) Vertreter: Schaller, Renate

(57) **Zusammenfassung**

Die Erfindung betrifft eine Kühlbox (1), umfassend einen mit einem trockenen Schutzgas befüllbaren Kühlbehälter, in dem ein mit röhrchenförmigen Gefäßen bestücktes Rack untergebracht ist. Der Kühlbehälter ist mit einem zu einem Deckelteil (7) gehörenden, verschiebbaren Deckel (12), bevorzugt gebildet durch einen Außendeckel (7.2) und einen in diesen integrierten, drehbaren Innendeckel (7.3), abgedeckt, wobei in jeder Position des Deckels (12) der Kühlbehälter vollständig abgedeckt ist, sodass ein eingefülltes Schutzgas nicht entweichen kann. Durch eine aufeinander abgestimmte Verschiebung des Außendeckels (7.2) und Verdrehung des Innendeckels (7.3) kann wenigstens eines der im Innendeckel (7.3) vorgesehenen Durchgangslöcher (7.3.1) über jeweils einem röhrchenförmigen Gefäß angeordnet werden, sodass durch dieses hindurch das fluchtend darunterstehende, röhrchenförmige Gefäß mittels einer handelsüblichen Pipettenspitze / Dispensiernadel eines Pipettierautomaten mit einer Probe befüllt werden kann.

## Beschreibung

Im Laboralltag werden regelmäßig Blutproben oder auch andere biologische Proben untersucht. Es ist von Interesse, diese Proben noch frisch zu untersuchen, aber auch zu einem späteren Zeitpunkt für andere oder vergleichende Untersuchungen erneut zu untersuchen. Häufig treffen die Proben über einen zu betrachtenden Zeitverlauf unregelmäßig ein (z. B. Notfallproben, überdurchschnittlich viele Proben nach der Morgenvisite, Einsendeproben), weshalb ein kontinuierliches Untersuchen der Proben in einem gleichen frischen Zustand meist nicht möglich ist. Es ist bekannt, dass die Qualität der Proben durch Lagerung bei Raumtemperatur leidet. D. h. Laborergebnisse, die an einer frischen Probe unmittelbar ermittelt wurden, stimmen nicht unbedingt mit denen der gleichen Probe nach längerer Lagerung überein. Wesentliche Informationen gehen durch unsachgemäße Lagerung verloren. Es ist bekannt, dass durch Kühlung der Proben, bzw. deren Lagerung bei tiefen Temperaturen, die Probenqualität aufrechterhalten werden kann. Beispiele hierfür sind die sogenannten Biobanken, in denen Proben bei Stickstofftemperatur über Jahrzehnte gelagert werden.

In der täglichen Routine durchlaufen mehrere Tausend Proben hochmoderne Laborautomaten. Mit Hilfe von Labormanagementsoftware werden diese Proben verwaltet, entsprechend den beauftragten Diagnosewünschen den entsprechenden Testgeräten zugeführt und die erhaltenen Testergebnisse archiviert und verschickt. Proben, die für eine spätere Verwendung vorgesehen sind, können problemlos aus diesem Prozess ausgeschleust werden. Es mangelt aber bislang an einer einfachen Möglichkeit, diese schnellstmöglich (vor allem aber auch automatisch) geeignet zu portionieren (aliquotieren) und dabei schnellstmöglich auf eine geeignete Lagerungstemperatur abzukühlen.

Üblicherweise erfolgt das Aliquotieren mit Hilfe von Pipettierautomaten, welche die mit Barcode etikettierten Entnahmeröhrchen, in denen die Proben im Labor eintreffen, erkennen (mit der Datenbank vergleichen), die Proben jeweils mit Hilfe von Pipettenspitzen oder Dispensiernadeln eines Pipettierautomaten aufnehmen (aufsaugen, aspirieren) und durch Verteilen auf mehrere für das Einfrieren geeignete, ebenfalls codierte, röhrchenförmige Gefäße in Aliquoten (Teilmengen der Probe) aufteilen. Verwendete Entnahmeröhrchen bekannter Hersteller sind z. B. Monovette® von Sarstedt oder Vacutainer® von BD.

Für die Aliquoten werden röhrchenförmige Gefäße (2D Barcoded CryoTubes), beispielsweise der Hersteller Thermo Matrix, Thermo Scientific, ABgene oder FluiX, genutzt. Diese wiederum stehen üblicherweise in Kunststoff-Racks (Tube Racks) in 8 mal 12er Anordnung (8 Zeilen, A-H und 12 Spalten, 1-12) zur Verfügung.

Oft handelt es sich bei den Aliquoten um 1 ml-Anteile des Serumüberstandes der Proben. Aber auch andere Bestandteile einer Blutprobe oder völlig andere biologische Proben (andere Körperflüssigkeiten bzw. gelöste biochemische Proben, auch unabhängig vom ggf. notwendigen Lösungsmittel) sind möglich.

Bezüglich der Verarbeitung und Lagerung von Proben werden vier Temperaturbereiche als praktikabel anwendbar betrachtet. Schnelle Verarbeitung flüssiger Proben bei Raumtemperatur, Zwischenlagerung bei -20°C für den Bereich von wenigen Stunden, Lagerung bei -80°C über viele Wochen und Monate sowie die unbegrenzte Lagerung unter echten Kryo-Bedingungen, also in flüssigem Stickstoff bei 77°K. Im Laboralltag erfolgt dies oft stufenweise. So erfolgt das Einlagern in den -80°C-Bereich eines automatischen Lagers oft über ein -20°C-Schleusensystem (z. B. Liconic ULT store). Eine erfindungsgemäße Kühlbox dient der -20°C-Zwischenlagerung und ist unabhängig von der weiteren Verwendung der Probe zu verwenden.

Nach derzeitiger Praxis werden die röhrchenförmigen Gefäße mit den im Labor häufig zeitlich unbestimmt eintreffenden Proben bei Raumtemperatur befüllt und damit wird ein Rack vollständig bestückt, bevor das vollständig gefüllte Rack in eine Kühleinrichtung gegeben wird, bzw. es wird in einer Kassette abgelegt, die eine Vielzahl von Plätzen für die Racks aufweist, und erst die vollständig mit Racks bestückte Kassette wird in eine Kühleinrichtung gegeben, wie z. B. in den STT3k0-DF der Firma Liconic AG. Die einzelnen Proben sind, bevor sie in die Kühleinrichtung kommen, unterschiedlich lange der Raumtemperatur ausgesetzt, was wie erläutert zu Qualitätsverlusten führt.

Der Erfindung liegt die Aufgabe zugrunde, eine Lösung zu finden, mit der auch zeit- und mengenmäßig unregelmäßig in einem Labor eintreffende Proben in kürzester Zeit auf eine Kühltemperatur abgekühlt werden können.

Die Aufgabe der Erfindung wird mit einer Kühlbox mit einem mit röhrchenförmigen Gefäßen bestückten Rack zum automatischen Befüllen mittels eines Pipettierautomaten gelöst. Diese Kühlbox weist ein Bodenteil und ein Deckelteil, ein mit röhrchenförmigen Gefäßen matrixförmig in Zeilen und Spalten bestücktes Rack, wobei die Zeilen über eine Breite und die Spalten über eine Länge des Racks verlaufen, einen von einer Wärmeisolation umgeschlossenen, wenigstens teilweise im Bodenteil angeordneten Kühlbehälter, der an die Außenmaße des bestückten Racks angepasst ist und in dem das bestückte Rack aufgestellt ist, auf.

Das Deckelteil besteht aus einem Deckelrahmen und einem im Deckelrahmen in Richtung der Spalten oder der Zeilen des Racks verschiebbaren Deckel, in dem eine solche Anzahl von Öffnungen vorgesehen sind, die an die Umfangsgröße einer handelsüblichen Pipettenspitzen/Dispensiernadel eines Pipettierautomaten angepasst sind, so dass über jedem einzelnen der im Rack stehenden, röhrchenförmigen Gefäße eine der Öffnungen positionierbar ist. Der Deckel deckt in jeder Position den Kühlbehälter vollständig ab, wodurch ein in den Kühlbehälter gefülltes trockenes Schutzgas, welches schwerer als Luft ist, in dem Kühlbehälter verbleibt. Die Kühlbox weist des Weiteren eine in der Wärmeisolation im Bodenteil integrierte und mit dem Kühlbehälter in Kontakt stehende Kühleinrichtung auf.

Vorteilhaft ist der Deckel aus einem Außendeckel mit einer wenigstens über die Ausdehnung einer Spalte reichenden Aussparung und einem gegenüber dem Außendeckel verstellbaren, die Aussparung abdeckenden Innendeckel gebildet. Dabei ist die wenigstens eine Öffnung ein Durchgangsloch im Innendeckel und durch die Verschiebung des Außendeckels und die Verstellung des Innendeckels zu den röhrchenförmigen Gefäßen positionierbar.

Es ist von Vorteil, wenn sich der Kühlbehälter anteilig im Bodenteil und anteilig im Deckelteil befindet, wobei der Anteil des Kühlbehälters im Deckelteil durch eine Ausformung in der Wärmeisolation und der Anteil des Kühlbehälters im Bodenteil durch eine Wanne gebildet wird. Für ein einfaches Einsetzen und Herausnehmen des Racks steht dieses vorteilhaft auf einem Tablett mit zwei Tablettgriffen. Für die Tablettgriffe sind in der Ausformung der Wärmisolation zwei gegenüberliegende Ausnehmungen vorgesehen, in denen die Tablettgriffe aufgenommen sind.

Zur Befüllung des Kühlbehälters ist es von Vorteil, im Bodenteil eine Zuführung für das Schutzgas vorzusehen. Die Kühleinrichtung besteht vorteilhaft aus einem plattenförmigen Peltierkühler und einem Flüssigkeits-Umlaufkühler, wobei der Peltierkühler einerseits unmittelbar mit der Wanne und andererseits mit dem Flüssigkeits-Umlaufkühler in Verbindung steht. Damit der Deckel in jeder Position den Kühlbehälter vollständig abgedeckt, ist dieser aus einer Mittellage heraus im positiven und negativen Richtungssinn jeweils um die halbe Länge des Racks zwischen zwei Endlagen verschiebbar und die Länge des Deckels ist entsprechend gewählt. Vorteilhaft ist der Innendeckel im Außendeckel integriert. Der Außendeckel und der Innendeckel können auch übereinander, sich überlappend, angeordnet sein.

In einer bevorzugten Ausführungsform der Kühlbox ist der Innendeckel rund und um den geometrischen Mittelpunkt des Außendeckels wenigstens um 180°drehbar und die Anzahl der Durchgangslöcher entspricht gleich der hälftigen Anzahl der in einer Zeile angeordneten röhrchenförmigen Gefäße, wobei die Durchgangslöcher auf einer Geraden radial zum Mittelpunkt des Außendeckels angeordnet sind. Um den Füllstand kontrollieren zu können, ist es von Vorteil, im Kühlbehälter einen Gassensor vorzusehen.

Nachfolgend soll die Kühlbox anhand von Ausführungsbeispielen unter Zuhilfenahme einer Zeichnung näher erläutert werden. Hierzu zeigen:
- Fig. 1a: ein erstes Ausführungsbeispiel für eine Kühlbox, bei dem sich der Deckel in einer Mittenlage befindet,
- Fig. 1b: die Kühlbox gemäß Fig. 1a, bei der sich der Deckel in einer von zwei Endlagen befindet,
- Fig. 2: Draufsicht auf eine Kühlbox ohne Deckelteil,
- Fig. 3a: Teilschnitt durch eine Seitenansicht einer Kühlbox,
- Fig. 3b: Teilschnitt durch die Draufsicht einer Kühlbox gemäß Fig. 1 a,
- Fig. 4: Bodenteil einer Kühlbox mit teilweise entfernter Wärmeisolation,
- Fig. 5: Deckelteil einer Kühlbox gemäß einem zweiten Ausführungsbeispiel in zwei verschiedenen Positionen,
- Fig.6: Deckelteil einer Kühlbox gemäß einem dritten Ausführungsbeispiel in zwei verschiedenen Positionen,
- Fig. 7: Deckelteil einer Kühlbox gemäß einem vierten Ausführungsbeispiel in zwei verschiedenen Positionen und
- Fig. 8: Deckelteil einer Kühlbox gemäß einem fünften Ausführungsbeispiel in zwei verschiedenen Positionen.

Eine erfindungsgemäße Kühlbox 1 zum automatischen Befüllen mittels eines Pipetterautomaten besteht im Wesentlichen aus einem mit röhrchenförmigen Gefäßen 2 bestückten Rack 3, einem mit einem trockenem Schutzgas 4 befüllbaren Kühlbehälter 5, einer Kühleinrichtung 10 sowie einem den Kühlbehälter 5 verschießenden, in einem Deckelrahmen 7.1 verschiebbaren Deckel 12.

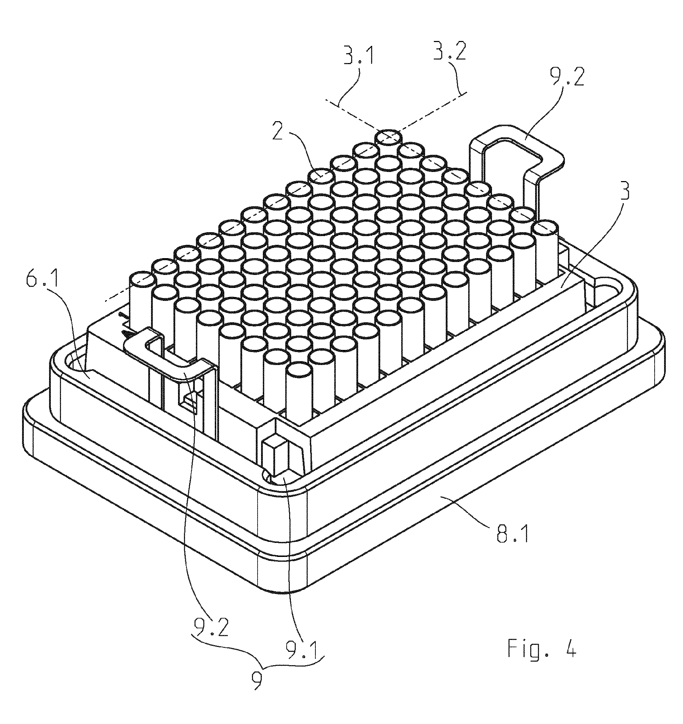

Bei dem mit röhrchenförmigen Gefäßen 2 bestückten Rack 3, gut zu sehen in Fig. 4, sind die röhrchenförmigen Gefäße 2 matrixförmig in Zeilen 3.1 und Spalten 3.2 angeordnet, wobei die Zeilen 3.1 über eine Breite und die Spalten 3.2 über eine Länge des Racks 3 verlaufen. Derartige Racks 3 sind aus dem Stand der Technik bekannt. Sie haben normierte Außenmaße und weisen üblicherweise in einer Zeile 3.1 acht und in einer Spalte 3.2 zwölf angeordnete Stellplätze für röhrchenförmige Gefäße 2 auf. Durch ihre Anordnung in Zeilen 3.1, denen in alphabetischer Reihenfolge jeweils ein Buchstabe (A...H) zugeordnet ist, und Spalten 3.2, denen aufeinanderfolgende natürliche Zahlen (1...12) zugeordnet sind, ist der Stellplatz eines jeden röhrchenförmigen Gefäßes 2 durch eine Reihe 3.1 und eine Spalte 3.2 eineindeutig adressiert, was die gezielte Positionierung eines später erläuterten Durchgangsloches 7.3.1 zum Durchführen einer Pipettenspitze oder Dispensiernadel eines Pipettierautomaten fluchtend über jedes der röhrchenförmigen Gefäße 2 erlaubt. Die Außenmaße eines mit röhrchenförmigen Gefäßen 2 bestückten Racks 3 werden in der Breite und Länge durch das Rack 3 selbst bestimmt. Die Höhe hingegen wird im Wesentlichen durch die Länge der röhrchenförmigen Gefäße 2 bestimmt, die allerdings auch in Normlängen zur Verfügung stehen.

Der Kühlbehälter 5 ist an die Außenmaße, Länge, Breite und Höhe des bestückten Racks 3 angepasst, das heißt, seine Innenmaße sind nur unwesentlich größer, um ein Verkannten des Racks 3 bei dessen Einsetzen und Herausnehmen aus dem Kühlbehälter 5 zu vermeiden. Vorteilhaft wird so der von dem Kühlbehälter 5 umschlossene Innenraum, der das zu kühlende Innenvolumen bestimmt, minimal gehalten. Wie einleitend erläutert wurde, soll die Kühlbox 1 für eine Kühlung von Proben bei -20°C verwendet werden. Der Kühlbehälter 5 ist durch den Deckel 12, hier gebildet durch einen Außendeckel 7.2 und einen integrierten Innendeckel 7.3, verschlossen, wobei im Innendeckel 7.3, wie später erläutert wird, wenigstens ein Durchgangsloch 7.3.1 vorgesehen ist. Um ein Eintreten von Luft durch dieses wenigstens eine Durchgangsloch 7.3.1 in den Kühlbehälter 5 und damit Vereisungen innerhalb des Kühlbehälters 5 zu verhindern, was eine Folge von Kondensatbildung und dessen nachfolgender Gefrierung ist, ist der Kühlbehälter 5 mit einem Schutzgas 4 gefüllt. Hierfür wird Schutzgas 4 verwendet, das schwerer als Luft ist, insbesondere Argon. Zum Befüllen ist an dem Kühlbehälter 5 eine Zuführung 4.1 vorgesehen, über die das Schutzgas 4 in den geschlossenen Kühlbehälter 5 gefüllt wird. Vorteilhaft ist im Kühlbehälter 5 ein Gassensor 11 vorhanden, der den Füllstand des Kühlbehälters 5 detektiert.

Obwohl das Innenvolumen des Kühlbehälter 5, wie an späterer Stelle erläutert wird, zumindest bei diesem ersten Ausführungsbeispiel, permanent über mindestens eine Öffnung 13, insbesondere ein Durchgangsloch 7.3.1, mit der Atmosphäre verbunden ist, kann aufgrund der Befüllung mit dem Schutzgas 4 keine Luft in den Kühlbehälter 5 eindringen bzw. ein Eindringen von Luft wird damit weitestgehend vermieden. Natürlich wäre es auch möglich, dieses wenigstens eine Durchgangsloch 7.3.1 grundsätzlich abzudecken und die Abdeckung nur zu entfernen, wenn durch das wenigstens eine Durchgangsloch 7.3.1 hindurch die röhrchenförmigen Gefäße 2 befüllt werden, wie an späterer Stelle erläutert wird. Eine solche Lösung bedeutet jedoch zusätzlichen mechanischen Aufwand und Steuerungsaufwand und vermeidet ein Eindringen von Luft bei Weitem nicht so gut, wie dies durch das Schutzgas 4 erreicht wird.

Damit das mit röhrchenförmigen Gefäßen 2 bestückte Rack 3 in den Kühlbehälter 5 eingesetzt werden kann, ist die Kühlbox 1 in ein Bodenteil 6 und ein abnehmbares Deckelteil 7 aufgeteilt, die miteinander verbunden, das heißt im geschlossenen Zustand der Kühlbox 1 den verschlossenen Kühlbehälter 5 integrieren.

Insbesondere für ein bequemes Handling des befüllten Racks 3 beim Einsetzen und Herausnehmen ist der Kühlbehälter 5 vorteilhaft nicht vollständig im Bodenteil 6 untergebracht und wird durch das Aufsetzen des Deckelteils 7 lediglich abgedeckt, sondern der Kühlbehälter 5 besteht aus einem Anteil im Deckelteil 5.1 und einem Anteil im Bodenteil 5.2. Dabei ist der Anteil des Kühlbehälters im Bodenteil 5.2, welcher einen Kühlbehälterboden und ca. 80 % der Höhe von vier Kühlbehälterseitenwänden umfasst, wenigstens teilweise als eine Wanne 6.1 ausgeführt. Die Wanne 6.1 ist aus einem gut wärmeleitenden Material mit einer hohen Wärmekapazität. Hier kommt bevorzugt Aluminium zum Einsatz, aber auch andere Metalle oder sogar moderne Kunststoffe können verwendet werden. Die Wanne 6.1 ist allseitig, vorteilhaft nur von einem ersten Teil einer Wärmeisolation 8 umgeben. Vorteilhaft ist dieser erste Teil der Wärmeisolation 8 ein erster Formkörper 8.1, dessen äußerer Umfang und Gestalt die Form und Größe des Bodenteils 6 der Kühlbox 1 prägen.

Um das Deckelteil 7 formschlüssig auf das Bodenteil 6 aufsetzen zu können, was eine definierte Positionierung zueinander gewährleistet, ragt der erste Formkörper 8.1 vorteilhaft über die Wanne 6.1 hinaus und ist nach außen hin umlaufend abgestuft, womit dieser vergleichsweise einen größeren freien Innenquerschnitt umschließt als die Wanne 6.1.

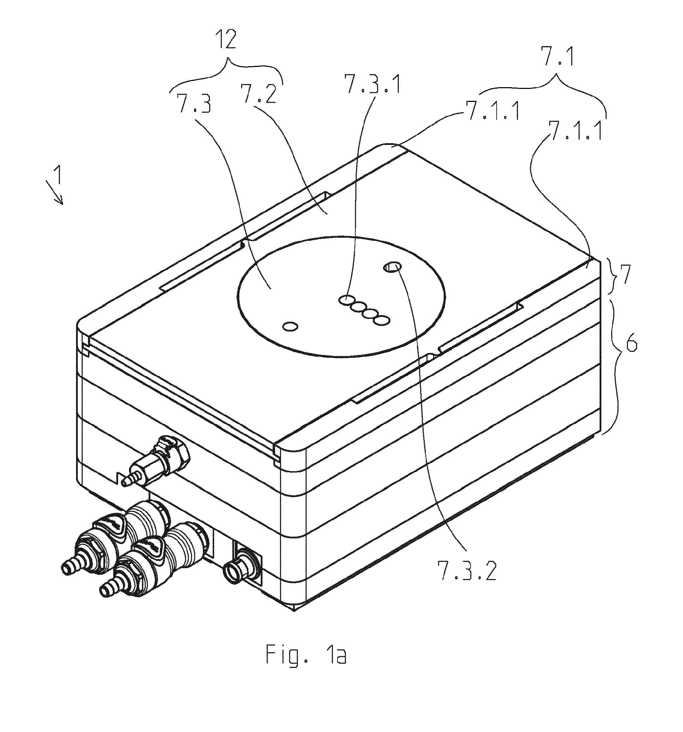

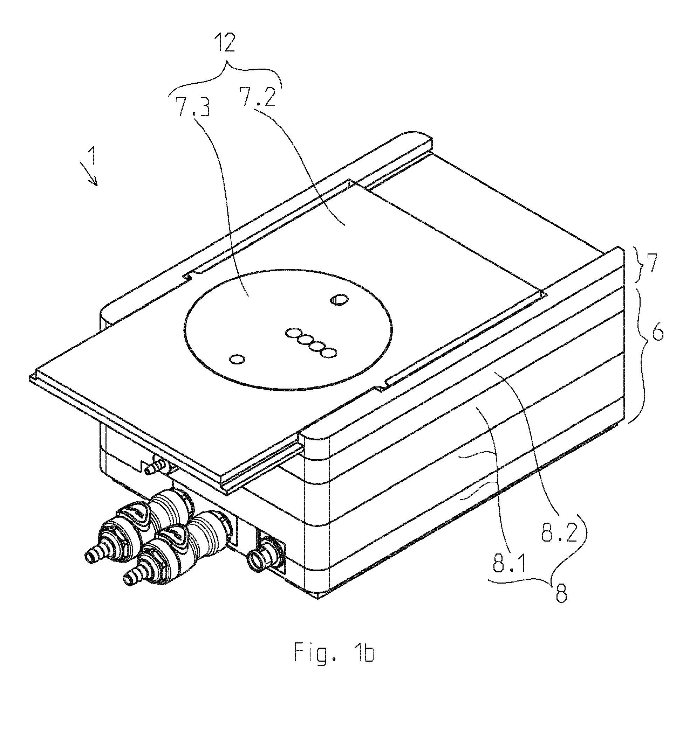

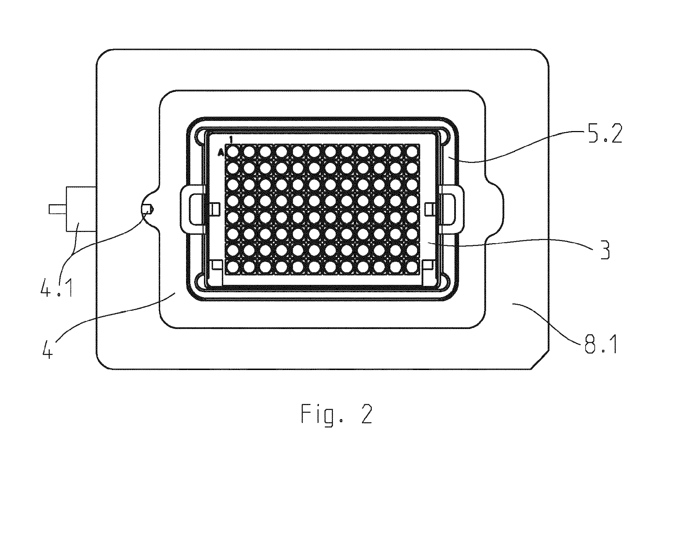

Das Deckelteil 7 umfasst im Wesentlichen einen Deckelrahmen 7.1, einen im Deckelrahmen 7.1 verschiebbaren Deckel 12, der wenigstens ein an die Umfangsgröße einer handelsüblichen Pipettenspitze/Dispensiernadel eines Pipetterautomaten angepasstes Durchgangsloch 7.3.1 aufweist. Gemäß einem ersten Ausführungsbeispiel, dargestellt in den Fig. 1 bis Fig. 4, ist der Deckel 12 aus einem Außendeckel 7.2 und einen im Außendeckel 7.2 integrierten, verdrehbaren Innendeckel 7.3 gebildet.

Der Deckelrahmen 7.1 ist an der dem Bodenteil 6 zugewandten Unterseite durch einen zweiten Teil der Wärmeisolation 8, der als ein zweiter Formkörper 8.2 ausgebildet ist, verstärkt. Die äußere Umfangsform und Umfangsgröße des zweiten Formkörpers 8.2 entspricht der äußeren Umfangsform und Umfangsgröße des ersten Formkörpers 8.1. Nach innen hin ist sie umlaufend so abgestuft, dass beim Aufsetzen des Deckelteils 7 auf das Bodenteil 6 zwischen Bodenteil 6 und Deckelteil 7 ein Formschluss entsteht. Der Anteil des Kühlbehälters im Deckelteil 5.1 wird durch eine Ausformung 8.3 in dem zweiten Formkörper 8.2 gebildet. Diese Ausformung 8.3 zeigt vorteilhaft einen freien inneren Querschnitt gleich dem Außenquerschnitt der Wanne 6.1. Vorteilhaft sind in dem zweiten Formkörper 8.2 zwei gegenüberliegende Ausnehmungen 8.4 vorgesehen, um Raum für zwei Tablettgriffe 9.2 eines Tabletts 9 zu schaffen.

Für ein bequemes und sicheres Handling des bestückten Racks 3, insbesondere beim Einsetzen und Herausnehmen aus der Kühlbox 1, ist dieses vorteilhaft auf einem Tablett 9 stehend in dem Kühlbehälter 5 untergebracht. Das Tablett 9 hat einen in seiner Größe an das Rack 3 angepassten rahmenförmigen Tablettboden 9.1 und seitliche Tablettgriffe 9.2.

Der Deckelrahmen 7.1 weist zwei parallel angeordnete Rahmenleisten 7.1.1 auf, zwischen denen der Außendeckel 7.2 in Richtung der Spalten 3.2 des befüllten Racks 3 aus einer Mittellage heraus, siehe Fig. 1a, im positiven und negativen Richtungssinn zwischen zwei Endlagen, siehe z. B. Fig. 1b, verschiebbar ist. Vorteilhaft ist der mögliche Verschiebeweg gleich der halben Länge des Racks 3. Die Länge des Außendeckels 7.2 ist so gewählt, dass auch in den Endlagen der Kühlbehälter 5 vollständig durch den Außendeckel 7.2 und den Innendeckel 7.3 abgedeckt bleibt.

Gemäß dem ersten Ausführungsbeispiel, gezeigt in den Fig. 1 - 4, ist der Innendeckel 7.3 rund und um den geometrischen Mittelpunkt des Außendeckels 7.2 innerhalb des Außendeckels 7.2 wenigstens um 180°drehbar. In dem Innendeckel 7.3 sind entlang einer radial zum Mittelpunkt verlaufenden Geraden eine Anzahl von Durchgangslöchern 7.3.1 vorgesehen, die gleich der hälftigen Anzahl der in einer Zeile 3.1 angeordneten röhrchenförmige Gefäße 2 entspricht. Bei einem Rack 3 mit 8 x 12 röhrchenförmigen Gefäßen 2 sind das entsprechend vier Durchgangslöcher 7.3.1. Ihr Abstand ist auf den Abstand der Symmetrieachsen der röhrchenförmigen Gefäße 2 abgestimmt, sodass die vier Durchgangslöcher 7.3.1 oberhalb jeweils entlang einer halben Zeile 3.1 angeordneter röhrchenförmiger Gefäße 2 zu diesen fluchtend positioniert werden können. Die Durchgangslöcher 7.3.1 sind vorteilhaft so klein wie möglich und so groß wie nötig, um handelsübliche Pipettenspitzen/Dispensiernadeln einführen zu können. Durch die Kombination der Verschiebung des Außendeckels 7.2 und der Drehung des Innendeckels 7.3 kann zu jedem röhrchenförmigen Gefäß 2 fluchtend ein Durchgangsloch 7.3.1 positioniert werden.

Um die Verschiebung und Verdrehung automatisieren zu können, ist vorteilhaft am Innendeckel 7.3 außermittig wenigstens ein Koppelelement 7.3.2 vorgesehen, über welches ein extern gesteuerter und angetriebener Greifer mit dem Innendeckel 7.3 verbunden werden kann. Über den verbundenen Greifer kann in den Inndeckel 7.3 unmittelbar eine Drehbewegung und in den Außendeckel 7.2 mittelbar eine Schubbewegung eingeleitet werden, womit das wenigstens eine Durchgangsloch 7.3.1 durch die Verschiebung des Außendeckels 7.2 und die Verstellung des Innendeckels 7.3 über jedem einzelnen der im Rack 3 stehenden röhrchenförmigen Gefäße 2 positionierbar ist. Der Greifer kann z. B. an einem einen größeren Laborbereich überspannenden Portal befestigt und gleichzeitig für mehrere erfindungsgemäße Kühlboxen 1 nutzbar sein.

Die Ansteuerung und die für die Bewegung entsprechenden Antriebe können auch an der Kühlbox 1 selbst vorgesehen sein. Dies würde jedoch die Kühlbox 1 wesentlich teurer, reparaturanfälliger, schwerer und unhandlicher machen. Grundsätzlich kann die Kühlbox 1 auch manuell bedient werden. Auch kann mittels eines am Pipettierautomaten vorgesehenen Schaftes, vorgesehen zur Aufnahme von Pipettenspitzen, die Positionierung des Innen- und des Außendeckels 7.3 und 7.2 erfolgen.

Die der Kühlbox 1 zugrundeliegende Idee erlaubt es, auch die Durchgangslöcher 7.3.1 so groß auszuführen, dass die röhrchenförmigen Gefäße 2 durch sie hindurch in die Kühlbox 1 eingeführt und an den einzelnen Stellplätzen gezielt in dem Rack 3 abgesetzt werden können. Eine derartige Ausführung und Verwendung der Kühlbox 1 hat allerdings den Nachteil, dass nicht nur die Probe, die in der Regel Raumtemperatur hat, in den Kühlbehälter 5 gefüllt und heruntergekühlt werden muss, sondern auch die röhrchenförmigen Gefäße 2. Die Abkühlung dauert daher länger und die Gefahr, dass über die Durchgangslöcher 7.3.1 das Schutzgas 4 teilweise entweicht und Luft eindringt, was insbesondere bei Erschütterungen des Kühlbehälters 5 und damit Verwirbelungen des Schutzgases 4 und Zugluft im Labor passieren kann, wird wesentlich größer. Man würde in diesem Fall auf wesentliche Vorteile verzichten, die eine erfindungsgemäße Kühlbox 1 bietet.

Indem nur Pipettenspitzen/Dispensiernadeln kurzzeitig und auch nur anteilig in den Kühlbehälter 5 eingeführt werden, ändert sich die im Kühlbehälter 5 herrschende Temperatur nicht bzw. sie ändert sich nicht messbar. Die Abgabe der Probe erfolgt in die auf die Wunschtemperatur der Abkühlung bereits vorgekühlten röhrchenförmigen Gefäße 2, sodass eine extrem schnelle Abkühlung der Probe innerhalb von wenigen Minuten stattfindet.

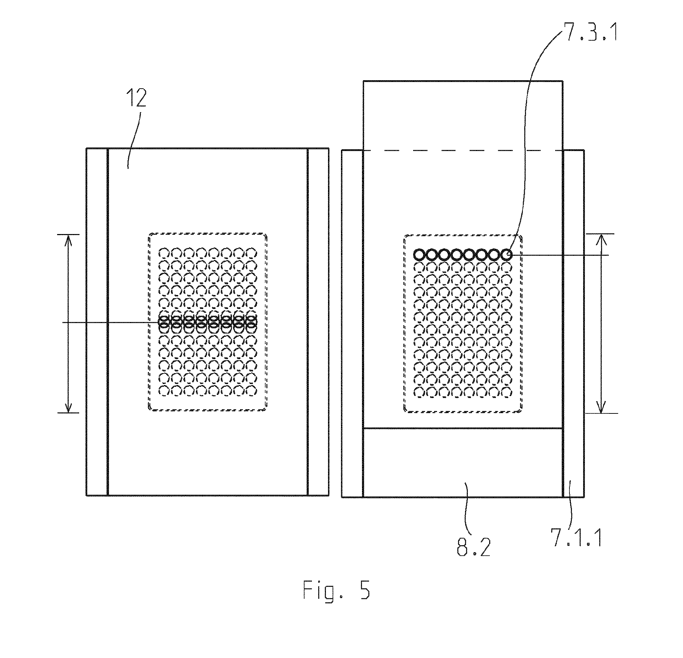

Nachfolgend werden verschiedene Ausführungsbeispiele für den Deckel 12 aufgezeigt. Als einfachste Variante ist der Deckel 12 gemäß einem zweiten Ausführungsbeispiel, gezeigt in Fig. 5, einteilig ausgeführt und weist eine Reihe von Durchgangslöchern 7.3.1 mit einer Anzahl gleich der Anzahl der Spalten 3.2 auf, z. B. 8, die auf einer Geraden senkrecht zur Verschieberichtung des Deckels 12 angeordnet sind. Als eine schlechtere Ausführung könnte stattdessen ein die Durchgangslöcher 7.3.1 ersetzender Schlitz vorgesehen sein. Der Deckel 12 ist aus einer Mittenlage heraus in zwei Richtungen bis jeweils in eine Endlage verschiebbar, womit die Reihe von Durchgangslöchern 7.3.1 nacheinander über allen Zeilen 3.1 positionierbar ist. Auch hier bleibt der Kühlbehälter 5, wie auch beim ersten Ausführungsbeispiel und den später noch beschriebenen Ausführungsbeispielen, mittels des Deckelteils 7 verschlossen. Vorteilhaft befindet sich die Reihe von Durchgangslöchern 7.3.1 in den Endlagen oberhalb des zweiten Formkörpers 8.2, womit in diesen Lagen der Kühlbehälter 5 dicht verschlossen ist, d. h. dass auch über die Durchgangslöcher 7.3.1 keine Verbindung mehr mit der Umgebung besteht. Der Deckel 12 und der zweite Formkörper 8.2 sowie der mögliche Verschiebeweg müssen in diesem Fall in Verschieberichtung länger ausgeführt werden als die Länge der Spalten 3.2.

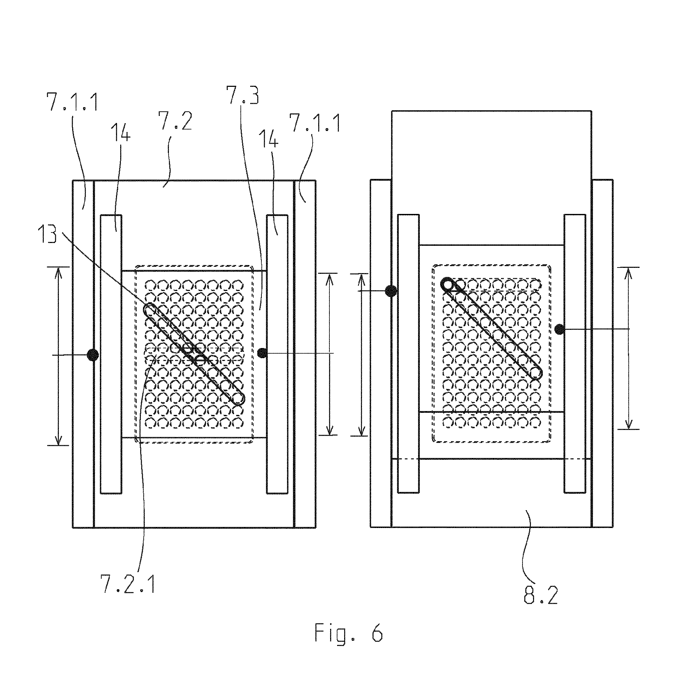

Gemäß einem dritten Ausführungsbeispiel, gezeigt in Fig. 6 kann der Innendeckel 7.3 auf dem Außendeckel 7.2, in Führungsleisten 14 geführt, in gleicher Richtung wie der Außendeckel 7.2 verschiebbar sein. Im Innendeckel 7.3 ist ein diagonal verlaufender Schlitz vorgesehen, der sich mit einer schlitzförmigen Aussparung 7.2.1 im Außendeckel 7.2 überlagert und eine in Richtung der Zeile 3.1 wandernde Öffnung 13 frei gibt. Anstelle des Schlitzes könnte vorteilhaft auch eine Reihe von Durchgangslöchern 7.3.1 im Innendeckel 7.3 vorgesehen sein.

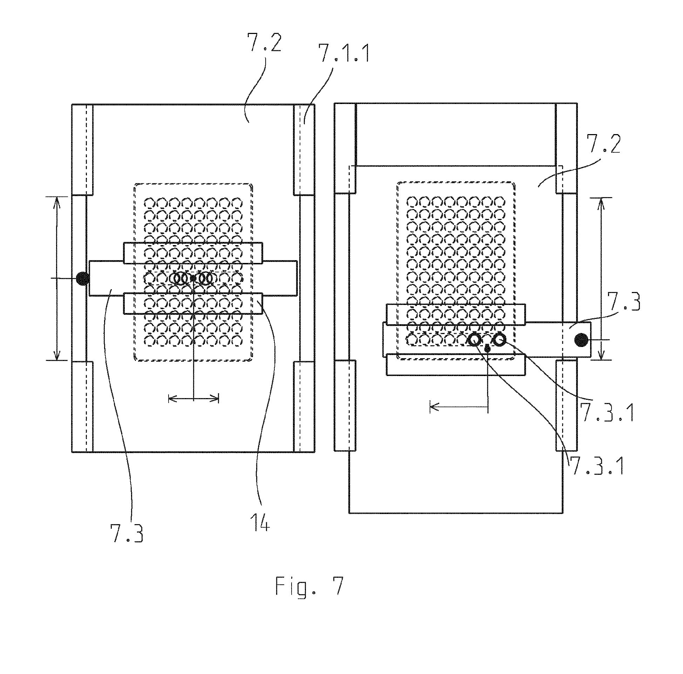

Nach einem vierten Ausführungsbeispiel, gezeigt in Fig. 7 ist der Innendeckel 7.3 auf dem Außendeckel 7.2 orthogonal zur Verschieberichtung des Außendeckels 7.2 in Führungsschienen 14 verschiebbar angeordnet. In dem Innendeckel 7.3 sind z. B. zwei Durchgangslöcher 7.3.1 vorgesehen.

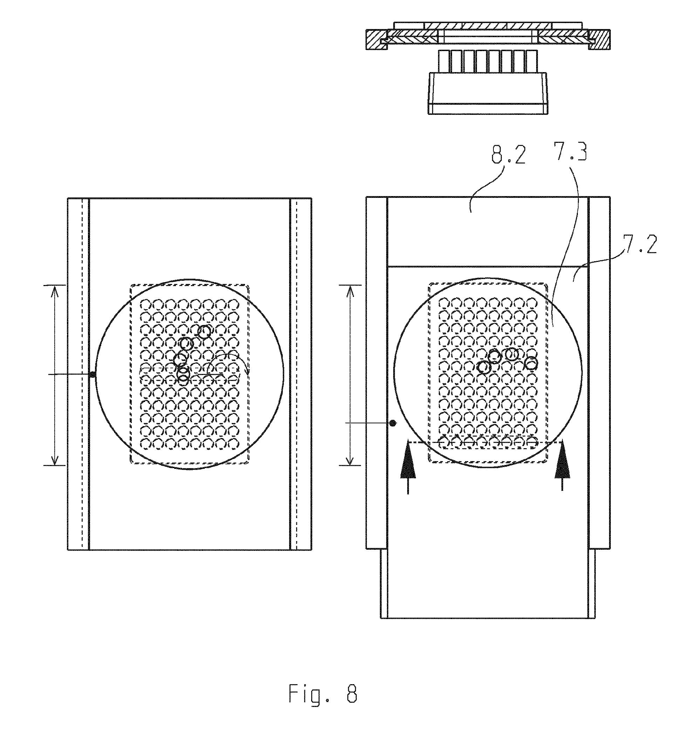

Bei einem fünften Ausführungsbeispiel, gezeigt in Fig. 8 ist der Innendeckel 7.3 drehbar auf den Außendeckel 7.2 aufgesetzt. Im Innendeckel 7.3 ist eine spiralförmige Anordnung von Durchgangslöchern 7.3.1 eingebracht, die jeweils bei Überlagerung mit einer schlitzförmigen Aussparung 7.2.1 im Außendeckel 7.2 eine Öffnung 13 bilden, die bei Verschiebung des Innendeckels 7.3 gegenüber dem Außendeckel 7.2 in Richtung der Zeile 3.1 auswandert. Anstelle der Anordnung von Durchgangslöchern 7.3.1 kann auch ein spiralförmiger Schlitz vorgesehen sein. Eine Vielzahl von weiteren Ausführungen sind möglich.

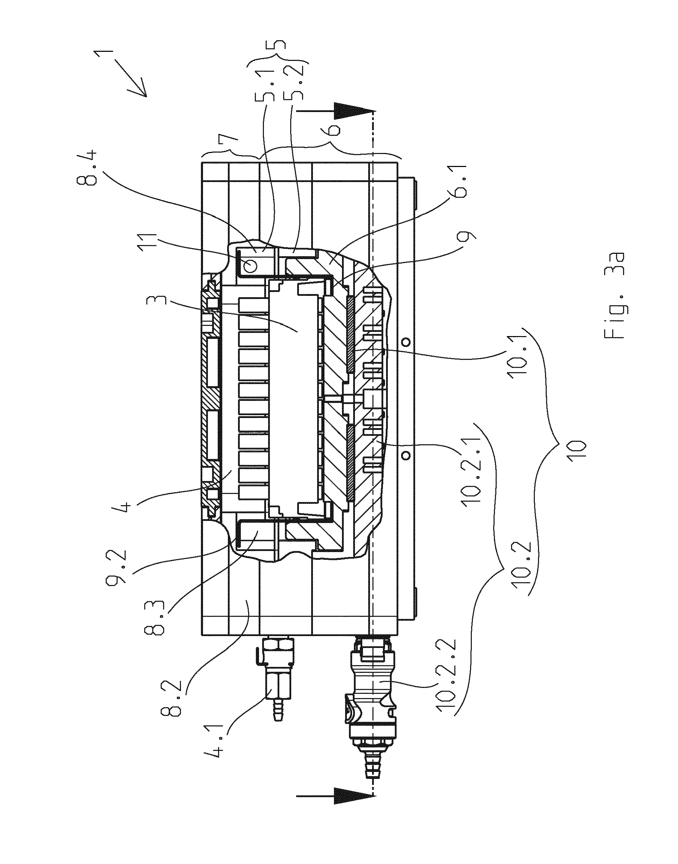

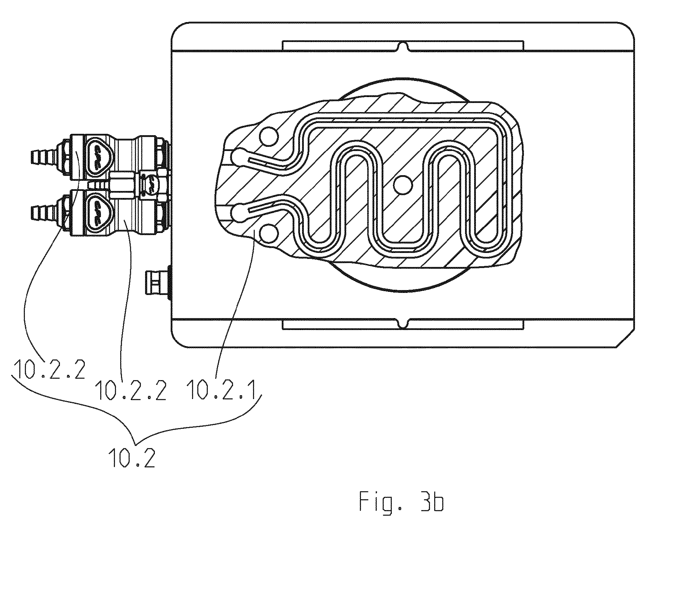

Die Kühleinrichtung 10, zu sehen in den Fig. 3a und 3b, umfasst einen Peltierkühler 10.1 und einen Flüssigkeits-Umlaufkühler 10.2 und ist in der durch den ersten Formkörper 8.1 gebildeten Wärmeisolation 8 so integriert, dass der Peltierkühler 10.1 mit einer seiner Seitenflächen großflächig mit dem Boden der Wanne 6.1 in Kontakt steht und sorgt so mit Hilfe eines in der Wanne 6.1 vorgesehenen Temperatursensors und der damit in Verbindung stehenden Regelelektronik für eine konstante Temperatur der Wanne 6.1. An der gegenüberliegenden (warmen) Seite des Peltierkühlers 10.1 grenzt der Flüssigkeits-Umlaufkühler 10.2 an und wirkt so als Wärmesenke für den Peltierkühler 10.1. Der Flüssigkeits-Umlaufkühler 10.2 umfasst eine Kühlplatte mit Kühlkanälen 10.2.1 und Anschlüsse 10.2.2, die mit einem Schlauchsystem an einen Wärmetauscher oder an ein Laborthermostat angeschlossen werden können. Stattdessen könnte die sogenannte warme Seite des Peltierkühlers 10.1 auch aus einer Kühlrippen-Lüfter-Kombination bestehen. Dies wäre aber für Laborautomaten bezüglich Verdunstung und Kontamination bzw. Geräuschpegel nicht günstig. Für den Transport des Kühlbehälters 5 sind an diesem vorteilhaft zwei abklappbare Henkel vorgesehen.

### Bezugszeichenliste

- 1: Kühlbox
- 2: röhrchenförmige Gefäße
- 3: Rack
- 3.1: Zeile
- 3.2: Spalte
- 4: Schutzgas
- 4.1: Zuführung
- 5: Kühlbehälter
- 5.1: Anteil des Kühlbehälters im Deckelteil 7
- 5.2: Anteil des Kühlbehälters im Bodenteil 6
- 6: Bodenteil
- 6.1: Wanne
- 7: Deckelteil
- 7.1: Deckelrahmen
- 7.1.1: Rahmenleisten
- 7.2: Außendeckel
- 7.2.1: Aussparung
- 7.3: Innendeckel
- 7.3.1: Durchgangsloch
- 7.3.2: Koppelelement
- 8: Wärmeisolation
- 8.1: erster Formkörper
- 8.2: zweiter Formkörper
- 8.3: Ausformung
- 8.4: Ausnehmung
- 9: Tablett
- 9.1: Tablettboden
- 9.2: Tablettgriff
- 10: Kühleinrichtung
- 10.1: Peltierkühler
- 10.2: Flüssigkeits-Umlaufkühler
- 10.2.1: Kühlplatte mit Kühlkanälen
- 10.2.2: Anschlüsse
- 11: Gassensor
- 12: Deckel
- 13: Öffnung
- 14: Führungsleisten

## Patentansprüche

1. Kühlbox (1) zum automatischen Befüllen mittels eines Pipettierautomaten, aufweisend
ein Bodenteil (6) und ein Deckelteil (7),
ein mit röhrchenförmigen Gefäßen (2) matrixförmig in Zeilen (3.1) und Spalten (3.2) bestücktes Rack (3), wobei die Zeilen (3.1) über eine Breite und die Spalten (3.2) über eine Länge des Racks (3) verlaufen,
einen von einer Wärmeisolation (8) umschlossenen, wenigstens teilweise in dem Bodenteil (6) angeordneten Kühlbehälter (5), der an die Außenmaße des bestückten Racks (3) angepasst ist und in dem das bestückte Rack (3) aufgestellt ist,
wobei das Deckelteil (7) einen Deckelrahmen (7.1) und einen im Deckelrahmen (7.1) in Richtung der Spalten (3.2) oder der Zeilen (3.1) des Racks (3) verschiebbaren Deckel (12), in dem Öffnungen (13), die an die Umfangsgröße einer handelsüblichen Pipettenspitze/Dispensiernadel eines Pipettierautomaten angepasst sind, in einer solchen Anzahl vorgesehen sind, dass über jedem einzelnen der im Rack (3) stehenden röhrchenförmigen Gefäße (2) eine der Öffnungen (13) positionierbar ist, wobei der Deckel (12) in jeder Position den Kühlbehälter (5) vollständig abdeckt, wodurch ein in den Kühlbehälter (5) gefülltes trockenes Schutzgas (4), welches schwerer als Luft ist, im Kühlbehälter (5) verbleibt,
sowie eine in der Wärmeisolation (8) im Bodenteil (6) integrierten und mit dem Kühlbehälter (5) in Kontakt stehenden Kühleinrichtung (10).

2. Kühlbox (1) nach Anspruch 1, **dadurch gekennzeichnet, dass** der Deckel (12) aus einem Außendeckel (7.2) mit einer wenigstens über die Ausdehnung einer Spalte (3.2) reichenden Aussparung (7.2.1) und einem gegenüber dem Außendeckel (7.2) verstellbaren, die Aussparung (7.2.1) abdeckenden Innendeckel (7.3) gebildet ist, wobei die wenigstens eine Öffnung ein Durchgangsloch (7.3.1) im Innendeckel (7.3) ist und durch die Verschiebung des Außendeckels (7.2) und die Verstellung des Innendeckels (7.3) zu den röhrchenförmigen Gefäßen (2) positionierbar ist.

3. Kühlbox (1) nach Anspruch 1, **dadurch gekennzeichnet, dass** sich der Kühlbehälter (5) anteilig im Bodenteil (6) und anteilig im Deckelteil (7) befindet, wobei der Anteil des Kühlbehälters im Deckelteil (5.1) durch eine Ausformung (8.3) in der Wärmeisolation (8) und der Anteil des Kühlbehälters im Bodenteil (5.2) durch eine Wanne (6.1) gebildet wird.

4. Kühlbox (1) nach Anspruch 1, **dadurch gekennzeichnet, dass** das befüllte Rack (3) auf einem Tablett (9) mit zwei Tablettgriffen (9.2) steht und in der Ausformung (8.3) der Wärmisolation (8) zwei gegenüberliegende Ausnehmungen (8.4) vorgesehen sind, in denen die Tablettgriffe (9.2) aufgenommen sind.

5. Kühlbox (1) nach Anspruch 1, **dadurch gekennzeichnet, dass** im Bodenteil (6) eine Zuführung (4.1) für das Schutzgas (4) vorgesehen ist.

6. Kühlbox (1) nach Anspruch 1 **dadurch gekennzeichnet, dass** die Kühleinrichtung (10) aus einem plattenförmigen Peltierkühler (10.1) und einem Flüssigkeits-Umlaufkühler (10.2) besteht, wobei der Peltierkühler (10.1) einerseits unmittelbar mit der Wanne (6.1) und andererseits mit dem Flüssigkeits-Umlaufkühler (10.2) in Verbindung steht.

7. Kühlbox (1) nach Anspruch 1 oder 2, **dadurch gekennzeichnet, dass** der Deckel (12) im Deckelrahmen (7.1) aus einer Mittenlage im positiven und negativen Richtungssinn jeweils um die halbe Länge des Racks (3) zwischen zwei Endlagen verschiebbar ist, wobei die Länge des Deckels (12) so gewählt ist, dass auch in den Endlagen der Kühlbehälter (5) vollständig durch den Deckel (12) abgedeckt bleibt.

8. Kühlbox (1) nach Anspruch 2, **dadurch gekennzeichnet, dass** der Innendeckel (7.3) im Außendeckel (7.2) integriert ist.

9. Kühlbox (1) nach Anspruch 2 oder 8, **dadurch gekennzeichnet, dass** der Innendeckel (7.3) rund ist und um den geometrischen Mittelpunkt des Außendeckels (7.2) wenigstens um 180° drehbar ist und die Anzahl der Durchgangslöcher (7.3.1) gleich der hälftigen Anzahl der in einer Zeile (3.1) angeordneten röhrchenförmigen Gefäße (2) entspricht, wobei die Durchgangslöcher (7.3.1) auf einer Geraden radial zum Mittelpunkt des Außendeckels (7.2) angeordnet sind.

10. Kühlbox (1) nach Anspruch 1, **dadurch gekennzeichnet, dass** im Kühlbehälter (5) ein Gassensor (11) vorhanden ist, um den Füllstand des Schutzgases (4) kontrollieren zu können.
